# FASCICULE DE BREVET EUROPEEN

(11) **EP 1 253 962 B1**
(45) Date de publication et mention de la délivrance du brevet: **14.01.2004**
(21) Numéro de dépôt: 01907763.5
(22) Date de dépôt: 09.02.2001
(51) Int. Cl.: A61M 25/01

(54) **ENSEMBLE COMPORTANT UNE PIECE POUR FACILITER L'ENFILAGE D'UN TUBE CATHETER SUR UN GUIDE**
EINHEIT MIT EINER VORRICHTUNG ZUM ERLEICHTERN DES AUFSCHIEBENS EINES KATHETERS AUF EINEN FÜHRUNGSDRAHT
ASSEMBLY COMPRISING A PIECE FOR SLIPPING ON OF A CATHETER TUBE ON A GUIDE

(30) Priorité: 09.02.2000 FR 0001586
(43) Date de publication de la demande: 06.11.2002
(73) Titulaire: VYGON, F-95440 Ecouen (FR)
(72) Inventeur: ROSSI, Daniel, F-95630 Meriel (FR)
(74) Mandataire: Schrimpf, Robert
(86) Numéro de dépôt international: PCT/FR2001/000397
(87) Numéro de publication internationale: WO 2001/058505

(56) Documents cités:
- EP-A- 0 328 760
- EP-A- 0 761 250
- EP-A- 0 801 955
- US-A- 5 320 613

## Description

La présente invention concerne une pièce de connection pour faciliter l'enfilage d'un tube cathéter sur un guide, notamment pour la mise en place d'un cathéter veineux central par la méthode de Seldinger, et ce dans un ensemble comportant une telle pièce.

La mise en place d'un cathéter veineux central par la méthode de Seldinger s'effectue comme suit :
1) Ponction de la veine avec une aiguille connectée à une seringue,
2) Après repérage du reflux sanguin, déconnexion de la seringue,
3) Introduction d'un guide métallique flexible dans la veine au travers de l'aiguille,
4) Retrait de l'aiguille en maintenant le guide,
5) Incision au point de ponction avec un scalpel pour faciliter le passage du cathéter (Fig.D),
6) Introduction dans la veine, par-dessus le guide métallique, d'un cathéter présentant une extrémité distale effilée,
7) Retrait du guide métallique.

En raison du faible diamètre du guide et de l'effilage de l'extrémité du cathéter, il est difficile et peu aisé d'enfiler le cathéter sur le guide.

La présente invention concerne une pièce de connection permettant de faciliter l'enfilage de l'extrémité distale effilée du cathéter sur l'extrémité proximale du guide.

La publication US 5 320 613 décrit différentes réalisations d'une pièce de connection destinée à constituer un chemin pour l'insertion d'un guide dans un tube cathéter. Cette pièce présente une portion distale conique pour recevoir et diriger le cathéter dans la pièce, une portion proximale opposée à la portion distale et également conique pour l'insertion du guide dans la pièce, ces deux portions communiquant par une portion intermédiaire qui débouche dans la portion distale par une colerette conique destinée à faciliter l'insertion du guide dans le cathéter ; dans une réalisation, la pièce présente une gorge longitudinale pour permettre la séparation latérale de la pièce après insertion du guide dans le cathéter.

Cette gorge qui est suffisamment large pour permettre le passage du tube cathéter pose un problème d'étanchéité et des moyens sont proposés dans cette publication pour assurer l'étanchéité de la jonction entre le cathéter et la pièce malgré la présence de la gorge.

La présente invention concerne une pièce conçue pour faciliter l'enfilage dans le cas où le cathéter présente une extrémité effilée, ce qui accentue la difficulté de l'insertion du guide dans le cathéter.

Selon l'invention, on utilise une pièce qui constitue un tunnel conformé intérieurement pour déterminer un premier et un deuxième cônes de passage, qui sont opposés par leurs sommets, qui communiquent entre eux et qui débouchent respectivement à l'une et à l'autre des deux extrémités du tunnel, le premier cône étant apte à recevoir l'extrémité effilée du cathéter jusqu'à ce que cette extrémité soit appliquée contre le sommet de ce cône et le deuxième cône étant tel que le guide puisse facilement être introduit dans le deuxième cône et guidé par la paroi de ce cône en direction du sommet du cône, ladite pièce étant apte à permettre la séparation latérale de la pièce du tube cathéter, cette pièce étant caractérisée par le fait que le premier cône épouse intérieurement la forme extérieure de l'extrémité du tube cathéter pour que la pièce puisse être tenue à friction sur cette extrémité, par le fait que les deux cônes communiquent par une ouverture correspondant à l'ouverture de l'extrémité effilée du tube cathéter, par le fait que la pièce présente une ailette latérale de préhension et une fente longitudinale située à l'opposé de cette ailette. La pièce est suffisamment souple pour que le tube cathéter puisse être séparé de la pièce après enfilage de ce tube sur le guide par passage à force du tube au travers de ladite fente dont les lèvres s'écartent en raison de la souplesse de la pièce.

La pièce de l'invention présente notamment les avantages suivants :
- facilité de fabrication en raison de sa forme simple,
- économie de matière en raison de sa structure,
- bonne tenue en main pour les manipulations d'enfilage et de séparation,
- possibilité de prémontage sur le tube cathéter,
- réduction des problèmes d'étanchéité.

On décrira ci-après un mode de réalisation d'une pièce conforme à la présente invention, en référence aux figures du dessin joint, sur lequel :
- la figure 1 est une perspective expliquant la manipulation à réaliser,
- la figure 2 est une vue à plus grande échelle d'un détail de la figure 1,
- la figure 3 est une vue analogue à celle de la figure 1 où la manipulation est facilitée par la pièce de l'invention,
- la figure 4 est analogue à la figure 2 mais avec la pièce conforme à l'invention,
- la figure 5 est une vue analogue de l'enfilage du cathéter sur le guide métallique spiralé,
- la figure 6 est une vue de détails correspondante analogue à celle des figures 2 et 4,
- la figure 7 est une vue analogue à celle de la figure 5 pendant la phase de séparation de la pièce et du cathéter, et
- la figure 8 est une vue de détails correspondante.

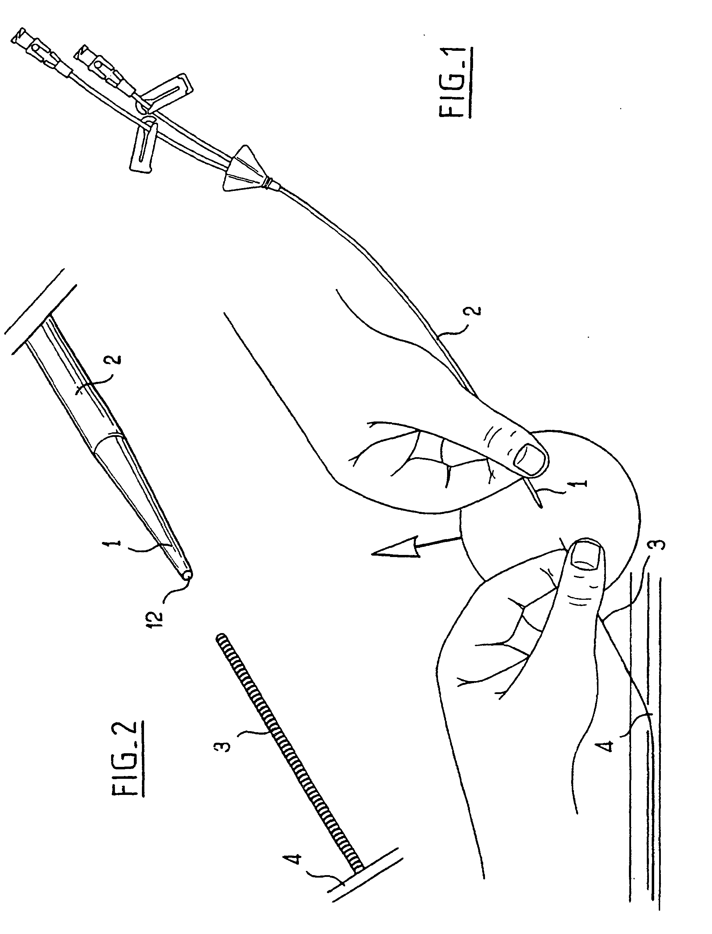

On a représenté sur la figure 1 une étape de la mise en oeuvre de la méthode de Seldinger dans laquelle il s'agit d'enfiler l'extrémité effilée (1) d'un tube cathéter (2) sur un guide spiralé souple (3) introduit dans une veine (4).

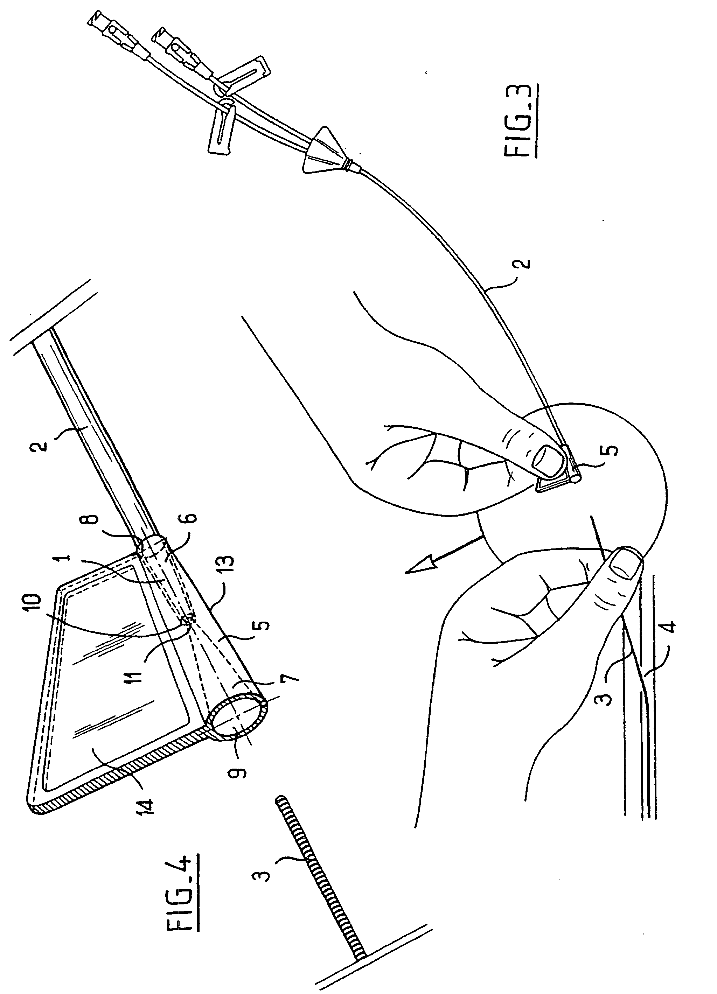

Pour faciliter cette introduction selon l'invention, on utilise une pièce (5) que l'on voit le mieux sur les figures 4, 6 et 8.

Dans une réalisation préférée, cette pièce est prémontée sur l'extrémité effilée (1) du tube cathéter (2) et le produit commercial est un ensemble qui comprend dans une pochette le cathéter et la pièce montée sur le cathéter, ainsi éventuellement que le guide.

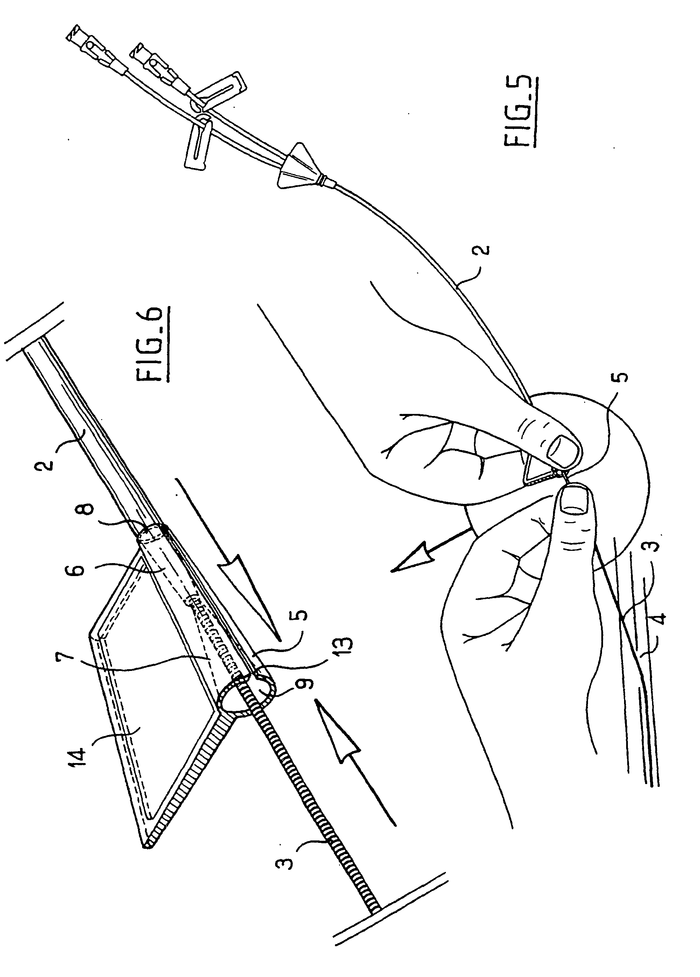

Cette pièce détermine un tunnel conformé intérieurement suivant un premier et un deuxième cônes de passage (6,7) qui sont opposés par leur sommet et qui débouchent par des ouvertures d'entrée respectives (8,9) aux deux extrémités du tunnel. Le premier cône (6) épouse intérieurement la forme extérieure de ladite extrémité effilée (1) tandis que le deuxième cône (7) présente une ouverture d'entrée (9) sensiblement plus large que le diamètre extérieur du guide spiralé (3). Les sommets (10,11) des deux cônes communiquent par une ouverture correspondant à l'ouverture (12) de l'extrémité effilée (1) du tube cathéter (2) en sorte que le guide métallique spiralé puisse être facilement introduit dans le deuxième cône (7) et guidé par la paroi de ce cône en direction du sommet du cône jusqu' à s'enfiler dans l'extrémité effilée (1) du tube cathéter (2) maintenue dans le premier cône par un léger serrage et appliquée contre le sommet de ce cône (fig.6)

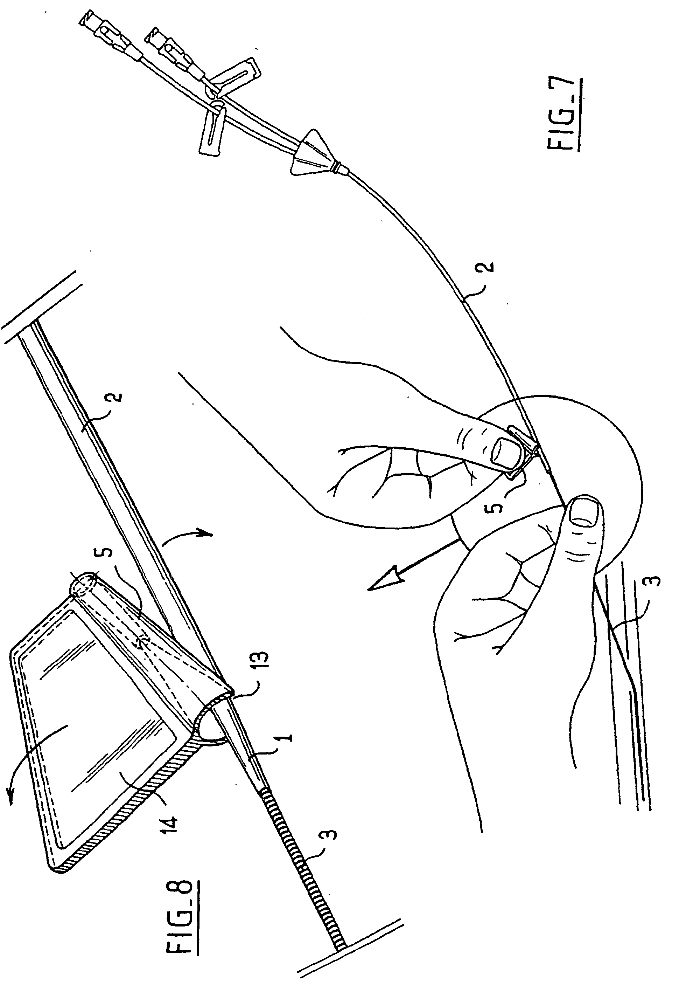

Selon l'invention, le tunnel a sa paroi qui présente une fente longitudinale (13), et la pièce (5) est en matériau relativement souple en sorte que le tube cathéter puisse être séparé de la pièce après enfilage de ce tube sur le guide spiralé, par passage à force du tube au travers de ladite fente dont les lèvres s'écartent en raison de la souplesse de la pièce (fig.8).

Selon une autre particularité de l'invention, la pièce comporte une ailette de préhension (14) sur un côté situé à l'opposé de ladite fente (13) pour faciliter les opérations d'enfilage et de séparation.

L'invention n'est pas limitée au mode de réalisation qui a été décrit, mais seulement par la portée des revendications.

## Revendications

1. Ensemble comportant un tube cathéter (2) et une pièce (5) pour faciliter l'enfilage de ce tube sur un guide (3), le tube cathéter (2) présentant une extrémité d'enfilage effilée (1), cette pièce constituant un tunnel conformé intérieurement pour déterminer un premier et un deuxième cônes de passage (6,7), qui sont opposés par leurs sommets, qui communiquent entre eux et qui débouchent respectivement à l'une et à l'autre (8,9) des deux extrémités du tunnel, le premier cône (6) étant apte à recevoir l'extrémité effilée du cathéter jusqu'à ce que l'extrémité soit appliquée contre le sommet de ce cône, et le deuxième cône (7) étant tel que le guide puisse facilement y être introduit et guidé par la paroi de ce cône en direction du sommet du cône, ladite pièce étant apte à permettre la séparation latérale de la pièce du tube cathéter, cette pièce étant **caractérisée par le fait que** le premier cône (6) épouse intérieurement la forme extérieure de l'extrémité du tube cathéter pour que la pièce puisse être tenue à friction sur cette extrémité, **par le fait que** les deux cônes communiquent par une ouverture correspondant à l'ouverture (12) de l'extrémité effilée du tube cathéter, **par le fait que** la pièce présente une ailette latérale de préhension (14) et une fente longitudinale (13) située à l'opposé de cette ailette, et **par le fait que** la pièce est suffisamment souple pour que le tube cathéter puisse être séparé de la pièce après enfilage de ce tube sur le guide par passage à force du tube au travers de ladite fente dont les lèvres s'écartent en raison de la souplesse de la pièce.

2. Ensemble selon la revendication 1 et qui comprend une pochette contenant le tube cathéter et ladite pièce.

3. Ensemble selon la revendication 2 dans lequel la pièce est prémontée sur le tube cathéter.

4. Ensemble selon la revendication 2 ou 3 et qui comporte également un guide apte à être enfilé dans le tube cathéter.

## Claims

1. Assembly comprising a catheter tube (2) and a piece (5) making it easier to engage this tube on a guide (3), the catheter tube (2) having a tapered engagement end (1), this piece constituting a tunnel which is shaped internally to determine first and second passage cones (6, 7) which are joined via their vertices, communicate with one another, and open out at respective ends (8, 9) of the tunnel, the first cone (6) being able to receive the tapered end of the catheter until the end is applied against the vertex of this cone, and the second cone (7) being such that the guide can be easily introduced into it and guided by the wall of this cone in the direction of the vertex of the cone, said piece being able to permit lateral separation of the piece from the catheter tube, this piece being **characterized in that** the inside of the first cone (6) matches the outer shape of the end of the catheter tube so that the piece can be held by friction on this end, **in that** the two cones communicate via an opening corresponding to the opening (12) at the tapered end of the catheter tube, **in that** the piece has a lateral grip tab (14) and a longitudinal slit (13) situated opposite this tab, and **in that** the piece is sufficiently flexible to ensure that the catheter tube can be separated from the piece, after engagement of this tube on the guide, by forcing the tube through said slit whose lips spread apart by reason of the flexibility of the piece.

2. Assembly according to Claim 1 and comprising a pouch containing the catheter tube and said piece.

3. Assembly according to Claim 2 in which the piece is already fitted on the catheter tube.

4. Assembly according to Claim 2 or 3 and also comprising a guide which can be engaged in the catheter tube.

## Patentansprüche

1. Einheit, umfassend einen Katheterschlauch oder ein Katheterrohr (2) und ein Teil (5) zur Erleichterung des Aufschiebens dieses Schlauchs oder Rohrs auf eine Führung (Führungsdraht) (3), wobei der Katheterschlauch bzw. das Katheterrohr (2) ein konisch zulaufendes Ende (1), an welchem das Aufschieben erfolgt, aufweist, wobei das Teil einen Tunnel bildet, welcher im Inneren geformt ist, so dass ein erster und ein zweiter Durchgangskegel (6,7) gebildet wird, die mit ihren Spitzen einander gegenüberliegen, die miteinander in Verbindung stehen und die jeweils an dem einen bzw. dem anderen (8, 9) der beiden Enden des Tunnels münden, wobei der erste Kegel (6) dazu geeignet ist, das konisch zulaufende Ende des Katheters aufzunehmen, bis das Ende an der Spitze dieses Kegels anliegt, und der zweite Kegel (7) derart ist, dass die Führung (Führungsdraht) leicht in diesen eingeführt und durch die Wand dieses Kegels in Richtung der Spitze des Kegels geführt werden kann, wobei das Teil dazu geeignet ist, die seitliche Trennung des Teils von dem Katheterschlauch bzw. -rohr zu erlauben, wobei dieses Teil **dadurch gekennzeichnet ist, dass** der erste Kegel (6) sich innerlich an die äußere Form des Endes des Katheterschlauchs bzw. -rohrs anschmiegt, so dass das Teil reibend auf diesem Ende gehalten werden kann, dass die beiden Kegel miteinander in Verbindung stehen durch eine Öffnung, welche der Öffnung (12) des konisch zulaufenden Endes des Katheterschlauchs bzw. -rohrs entspricht, dass das Teil einen seitlichen Flügel zum Ergreifen (14) und einen längs verlaufenden Schlitz (13), welcher diesem Flügel gegenüberliegt, aufweist und dass das Teil ausreichend flexibel ist, damit der Katheterschlauch bzw. das Katheterrohr von dem Teil nach dem Aufschieben dieses Schlauchs bzw. Rohrs auf die Führung getrennt werden kann durch durch Kraftaufwand erfolgendes Hindurchtreten des Schlauchs bzw. Rohrs durch den Schlitz hindurch, dessen Lippen sich aufgrund der Flexibilität des Teils auseinanderbewegen.

2. Einheit nach Anspruch 1 und welche eine Hülle, die den Katheterschlauch bzw. das Katheterrohr und das Teil enthält, umfasst.

3. Einheit nach Anspruch 2, bei welcher das Teil auf dem Katheterschlauch bzw. -rohr vormontiert ist.

4. Einheit nach Anspruch 2 oder 3 und die gleichfalls eine Führung umfasst, welche dazu geeignet ist, in den Katheterschlauch bzw. das Katheterrohr eingeschoben zu werden.
